# EUROPEAN PATENT APPLICATION

(11) **EP 4 233 833 A1**
(43) Date of publication of application: **30.08.2023**
(21) Application number: 22159101.9
(22) Date of filing: 28.02.2022
(51) Int. Cl.: A61K 8/35, A61K 8/40, A61K 8/41, A61K 8/49, A61K 31/11, A61K 31/121, A61K 31/14, A61K 31/15, A61K 31/352, A61K 31/445, A61K 31/4965, A61P 17/00, A61P 17/12, A61P 35/00, A61Q 19/08, A61K 8/365

(54) **ACTIVE AGENT MODULATING THE ACTIVITY OF AN ION CHANNEL FOR USE IN INHIBITION OF SKIN AGING**

(71) Applicant: CUTANEON - Skin & Hair Innovations GmbH, 22339 Hamburg (DE)
(72) Inventor: PAUS, Ralf, 22339 Hamburg (DE); BÍRÓ, Tamás, 4032 Debrecen (HU); MARDARYEV, Andrei, St Saviour, Jersey (GB)
(74) Representative: WSL Patentanwälte Partnerschaft mbB

(57) **Abstract**

In order to provide new active agents for treating skin aging in subjects without undesired side effects the present invention discloses active agents activating, enhancing, or positively modulating the cellular response of the transient receptor potential ion channel TRPM5 or increasing the expression of said ion channel.

## Description

The present invention is directed to an active agent for use in inhibition of skin aging, particularly for use in the treatment of premature or pathological skin aging conditions, wherein said active agent modulates the activity of an ion channel, including but not limited to activation, enhancement or positive modulation of the cellular response induced by an ion channel or increases the expression of said ion channel.

Further, the present invention is directed to a composition for use as a cosmetic or medicament in the treatment of premature or pathological skin aging conditions said composition comprising at least one active agent that modulates the activity of an ion channel, including but not limited to activation, enhancement or positive modulation of the cellular response induced by an ion channel or increases the expression of said ion channel.

In addition, the present invention is directed to a non-therapeutic method of inhibition of skin aging, wherein an effective amount of at least one active agent that modulates the activity of an ion channel, including but not limited to activation, enhancement or positive modulation of the cellular response induced by an ion channel or increases the expression of said ion channel, is administered to a subject.

Skin, the largest organ of the human body, establishes the barrier that segregates and protects the body from the environment. As all other organs, the skin also exhibits a continuous life-long and time-dependent aging process that is controlled by numerous intrinsic and extrinsic factors. Intrinsic aging, that is mostly visible in the sun-unexposed areas (e.g. the inner side of the arm), is mainly due to intrinsic genetic factors as well as the age-dependent perturbation of tissue and cell metabolism. Extrinsic aging, however, is induced and enhanced by a plethora of environmental factors such as chemical exposure (e.g. air pollution, improper use of skin-acting agents), smoking, poor or impaired nutrition, etc. Most importantly, the primary factor of extrinsic skin aging is the chronic, life-long exposure to solar ultraviolet (UV) radiation, i.e. photoaging.

Skin aging is characterized by multiple phenomenological and morphological changes such as thin, dry, fragile and sensitive skin, fine or course wrinkles, gradual atrophy that affects both the epidermis and the dermis, loss of elasticity and laxity, decrease of skin water content, rough texture, impaired and delayed wound regeneration, etc. These alterations are determined by specific changes in the expression and/or activities of various molecules and signaling pathways, induced by intrinsic or extrinsic pro-aging factors.

Among the increasing number of "aging markers", skin aging at the cellular and molecular level is characterized by, for example, thinning of the epidermis, decreased proliferation of the epidermal keratinocytes, decreased size of the epidermal stem cell pool that results in impaired regeneration capacity, degradation (hence decreased content) of fibrous and non-fibrous extracellular matrix components (including, but not limited to, collagens, elastins, fibrillins, and oligosaccharides), and alterations in expressions/activities of molecules of cellular metabolism, survival, stress resistance, and inflammation. Further, these "aging markers" are routinely assessed in pre-clinical and clinical settings when the efficacy and applicability of novel anti-aging actives are tested.

Importantly, skin aging, especially when premature or pathologically accelerated, does not only constitute an obvious cosmetic problem but also results in the premature development and/or augmentation of a plethora of skin diseases that require immediate and definite dermatological treatment. These include, but not limited to, rosacea (redness, pimples, swelling, and small and superficial dilated blood vessels affecting especially the face), asteatotic eczema (characterized by dry, itchy, and cracked skin), nummular eczema (features coin-shaped itchy, reddened patches), seborrheic dermatitis, skin infections, actinic keratosis (precancerous skin lesion linked to chronic UV irradiation), and benign (non-cancerous) as well as malignant (basal cell carcinoma, squamous cell carcinoma) skin tumors. Therefore, proper management of skin aging (especially of the premature and pathologically accelerated one), usually by cosmetic actives and formulations and medical devices, has an obvious dermatological benefit.

Currently, there are multiple actives on the market that are claimed to be instrumental for the management of skin aging. Several of said actives belong to the group antioxidants and include certain vitamins (e.g. vitamin C, alpha tocopherol or vitamin E, niacinamide), terpenes, and polyphenols. Since skin aging, by far, is a much more complex phenomenon that to be simplified to the level of impaired antioxidant properties (induced by intrinsic or extrinsic factors), the disadvantages of said antioxidants are that they do not specifically, or only marginally, target the cellular and molecular processes of skin aging, and hence their anti-aging effects are limited.

In addition, certain derivatives of vitamin A, i.e. retinoids, were also claimed to be effective in the management of skin aging. However, their widespread use is usually hampered by their extensive chronic adverse effects (e.g. irritation, redness, dryness, peeling, augmentation of eczema, increase photosensitivity to UV light) and there are severe safety concerns as retinoids were shown to cause DNA damage and hence may increase the risk of carcinogenesis.

In light of the above, there is a continuous need for active agents for actively controlling and/or treating skin aging in subjects without undesired side effects.

"Ion channels" are pore-forming proteins located in biological membranes. Through the pores of said proteins ions may pass the membrane down their electrochemical gradient. By either opening or closing the pore ion channels are capable of gating and controlling the flow of ions across the membranes thereby modulating the intracellular concentrations of ions. Alteration of intracellular ion concentrations affect a plethora of cellular responses and processes including, but not limited to, e.g. growth, differentiation, survival, death, mediator release, immune mechanisms, etc.

"Transient receptor potential channels" (TRP channels) are a group of ion channels located mostly on the plasma membrane, and there are about 30 TRP channels including TRPC, TRPV, TRPM, TRPN, TRPA, TRPP and TRPML. Said ion channels have a relatively non-selective permeability to cations, including sodium, calcium and magnesium.

"TRPM5" is the official gene symbol for "transient receptor potential cation channel subfamily M member 5" and identifies the protein that is encoded by the TRPM5 gene in humans (NCBI Gene ID: 29850; HGNC: 14323; NCBI mRNA sequence: NM_014555.3; NCBI protein sequence: NP_055370.1; Status on June 7, 2020).

The inventors of the present application have found that the naturally occurring pheromone 2,5-dimethylpyrazine (in the following referred to as "DMP"), being an agonist of the transient receptor potential ion channel TRPM5, causing the channel to be open, exerts a robust anti-aging effect.

Indeed, when topically or systemically applied in human *ex vivo* skin organ cultures or in *in vitro* cultures of human epidermal keratinocytes - complementary models that are suitable to assess multiple hallmarks of the skin aging process -, DMP upregulated expressions of multiple "skin aging markers" whose levels were found to be downregulated in aging skin. Specifically, as revealed by quantitative immunohistomorphometry and, in certain cases, gene expression profiling, DMP treatment increased expressions of Collagen 17A (an epidermal basal membrane and extracellular matrix component), Cytokeratin 15 (a marker of epidermal stem cells and proliferation), Ki67 (a nuclear non-histone protein that is universally expressed among proliferating cells), Phospho-Histone H3 Ser10 (pH3S10) (phosphorylation at Ser10 of histone H3 is tightly correlated with chromosome condensation during mitosis and cell proliferation), Lamin B1 (a regulator of cell survival, senescence, and aging), peroxisome proliferator-activated receptor-coactivator-1 α (PGC1a, a transcriptional coactivator that regulates the genes involved in energy metabolism and mitochondrial biogenesis), and Sirtuin-1 (a NAD-dependent protein deacetylase that links transcriptional regulation directly to intracellular energetics, DNA damage, metabolism, apoptosis and autophagy).

*Vice versa,* inhibition of TRPM5 using the selective inhibitor triphenylphosphine oxide (TPPO) consistently prevented the anti-aging effects of DMP.

In addition to the above active agents, the inventors identified a number of additional active agents acting on TRPM5 and, thus, being suitable for use in the treatment of skin aging.

Concluding, the present invention is characterised by an active agent for use in the treatment of skin aging, wherein said active agent activates, enhances, positively modulates the cellular response induced by the transient receptor potential ion channel TRPM5 or increases the expression of said ion channel.

According to one alternative of the present invention the active agent that activates, enhances, positively modulates the cellular response induced by the transient receptor potential ion channel TRPM5 is an agonist of said ion channel.

As referred to herein an "agonist" of the transient receptor potential ion channel TRPM5 is a substance that binds to said ion channel and activates or enhances the ion channel thereby augmenting the cellular response linked to TRPM5.

With respect to the transient receptor potential ion channel TRPM5 of the present invention the term "cellular response" is primarily to be understood as a change of ion concentration in the cell being the result of an agonist binding to the transient receptor potential ion channel TRPM5. However, the term also encompasses instances, where the binding of an agonist induces a cellular response without altering the intracellular ion concentration.

Accordingly, an inventive agonist of the transient receptor potential ion channel TRPM5 activates or enhances said ion channel to produce the cellular response like an endogenous agonist does.

In those embodiments, where the active agent is an agonist of the transient receptor potential ion channel TRPM5, said active agent is used in the treatment of skin aging and associated skin conditions.

In specific embodiments of the invention a TRPM5 agonist selected from the group consisting of dimethylpyrazine, dimethylethylpyrazine, tetramethylpyrazine, 2-heptanone, eugenol, SID2848719 (CAS number 702636-90-6, SMILES, NC(=O)C1(CCN(CC1)S(=O)(=O)c1ccc2OCCCOc2c1)N1CCCCC1), rutamarin, bergapten, xanthotoxin, isopimpinellin, carbachol, 3-deoxyglucosone, glucagon-like peptide 1, (E)-N-(3,4dimethoxybenzylidene)-2-naphthalene-1-yl)acetohydrazide, or a combination thereof.

Even though the inventors are not aware of any such instance, it cannot be excluded, that there exist prior art skin aging treatment compositions comprising one of the specific inventive active agents mentioned above as an ingredient, without attributing said ingredient any effect on TRPM5. In this event, such accidentally anticipated active agent shall be excluded from the scope of the invention by a corresponding disclaimer. Except for excluding said accidentally anticipated specific active agent said disclaimer shall not further affect the scope of the claims.

Non-limiting examples for active agents being accidentally anticipated in the prior art are
- chemical compounds being attributed no medical, pharmacological or biological activity at all by the corresponding prior art,
- chemical compounds being attributed another medical, pharmacological or biological activity by the corresponding prior art as compared to the herein disclosed specific inventive active agents, and/or
- chemical compounds being attributed the same medical, pharmacological or biological activity by the corresponding prior art as compared to the herein disclosed specific inventive active agents, but not being directed to TRPM5 but being directed to another target (e.g. receptor, enzyme, hormone, metabolite).

Alternatively, the TRPM5 agonist is an aptamer binding to the TRPM5 ion channel and activating or enhancing the ion channel to produce the cellular response.

The term "aptamer" as used herein refers to DNA, RNA or XNA oligonucleotide or peptide molecules that bind to a specific target molecule, such as ion channel molecules.

According to the present invention one of the above-mentioned active agents is used in the treatment of skin aging. In specific embodiments of the invention the treatment is effected locally, i.e. in, on or at the skin area to be treated. In some embodiments of the invention said treatment is effected topically, wherein the term "topical" refers to a formulation that is applied to a particular place on the skin. In specific embodiments the topical application is epicutaneous, meaning that the agonist is applied directly to the skin. In other embodiments of the invention the treatment is effected transdermally, wherein the term "transdermal" refers to a formulation that is applied across the Stratum corneum into the deeper skin layers, e.g. by injection with a standard needle or microneedles. In other embodiments of the invention the treatment is effected transappendageally, wherein the term "transappendageal" refers to an applied formulation that is permeating the skin via skin appendage structures (such as the hair follicles, sebaceous glands, and sweat glands) into the deeper skin layers.

The term "treatment" as used herein refers to any action resulting in the change of a physical condition. Particularly, the "treatment of skin aging" refers to any change of an initial skin condition, including, but not limited to, thin, dry, fragile and sensitive skin, fine or course wrinkles, gradual atrophy that affects both the epidermis and the dermis, loss of elasticity and laxity, decrease of skin water content, rough texture, impaired and delayed wound regeneration. Further, the "treatment of skin aging" also refers to prevention or treatment of aging-associated skin diseases including, but not limited to, rosacea (redness, pimples, swelling, and small and superficial dilated blood vessels affecting especially the face), asteatotic eczema (characterized by dry, itchy, and cracked skin), nummular eczema (features coin-shaped itchy, reddened patches), seborrheic dermatitis, skin infections, actinic keratosis (precancerous skin lesion linked to chronic UV irradiation), and benign (non-cancerous) as well as malignant (basal cell carcinoma, squamous cell carcinoma) skin tumors.

In specific embodiments of the invention the above-mentioned active agent is used as a cosmetic in the treatment of skin aging. Particularly, the use as a cosmetic occurs non-therapeutically, but in order to maintain an initial skin condition, such as young or less aged skin, wherein said initial condition is not caused by a disease or disorder.

In those embodiments, where the above mentioned active agent is used as a cosmetic the active agent used should be cosmetically acceptable, wherein "cosmetically acceptable" means that the active agent should not be toxic or harmful or have any other detrimental side effects upon application on skin.

In other specific embodiments of the invention the above mentioned active agent is used as a medicament in the topical treatment of skin aging-associated disorder, wherein the term "disorder" refers to any functional abnormality or disturbance of the normal healthy condition, and the term "medicament" refers to a substance useful in curing, treating or preventing a condition of disorder.

In those embodiments, where the above mentioned active agent is used as a medicament the active agent used should be pharmaceutically acceptable, wherein "pharmaceutically acceptable" means that the active agent should not be toxic or harmful or have any other detrimental side effects upon application on skin.

In some embodiments at least one of the inventive active is used as an ingredient of a composition for use as a cosmetic or medicament in the topical treatment of skin aging said composition further comprising at least one auxiliary agent selected from the group consisting of carriers, excipients, adjuvants, diluents, and disintegrants.

In specific embodiments of such compositions the auxiliary agent is selected from the group consisting of liposomes, nanoparticles, carboxymethyl cellulose, hydroxyethyl cellulose, mineral oil, petrolatum, glycerin, polysorbate 80, hydroxyethyl starch, dextran, and polyethylene glycol.

In the inventive compositions the concentration of the active agent usually is in the range of from 1 nM to 10.000 µM. In some embodiments the lower limit for the concentration of the active agent is 30 µM or even 100 µM. In some embodiments the upper limit is 3.000 µM or 1.000 µM. This results in preferred ranges of e.g. from 30 to 10.000 µM, from 30 to 3.000 µM, from 10 nM to 3.000 µM, 100 to 3.000 µM and the like.

Referring to the total weight of an inventive composition the concentration of the active agent may vary in specific embodiments within the range of from 0.1 to 30 wt.-%. In some embodiments the lower limit is 5 wt.-% or even 10 wt.-%. In some embodiments the upper limit is 25 wt.-% or 20 wt.-%. This results in preferred ranges of e.g. from 5 to 30 wt.-%, from 5 to 25 wt.- %, from 10 to 25 wt.-%, 10 to 30 wt.-% and the like.

In specific embodiments such compositions further comprise at least one other active agent being effective in the treatment of skin aging.

In such embodiments the other active agent may be selected from one of the prior art skin anti-aging agents, such as the ones that were mentioned in the introduction.

Generally, the inventive composition may be used in any formulation suitable for treatment of skin aging. In specific embodiments of the invention the composition is formulated in the form of an ointment, a lotion, a cream, a gel, a spray, a plaster or a sustained release plaster. In other specific embodiments of the invention the composition is formulated in the form of a solution. Said solution may be applied by means of a microneedle device or prior to sonication, electrical stimulation, etc.

As mentioned above, the inventive use of the above mentioned active agent may also occur non-therapeutically, wherein non-therapeutically refers to a treatment not being directed to curing, treating or preventing a condition of disorder (see above).

Therefore, the present invention is further directed to a non-therapeutic method of skin aging regulation, wherein an effective amount of at least one of the above mentioned active agent is administered to a subject.

The non-therapeutic method also encloses embodiments, where the above mentioned active agent is administered to the subject to be treated simultaneously, sequentially or separately together with at least one other active agent useful in the treatment of skin aging (see above).

The following examples show some of the features of specific embodiments of the present invention. However, the skilled reader will understand that those embodiments are just exemplary but do not restrict the inventive idea to exactly the features or the combination of features of the embodiments of the examples.

In the description of the examples it is referred to the following figures, wherein
- Figure 1: comprises two microscopic images and a bar graph showing that 2,5-dimethylpyrazine (DMP) increases epidermal expression of Collagen 17A, an epidermal basal membrane and extracellular matrix component, and whose level was shown to decrease with skin aging, in human skin organ culture,
- Figure 2: comprises two microscopic images and a graph showing that 2,5-dimethylpyrazine (DMP) increases epidermal expression of Cytokeratin 15, a marker of epidermal stem cells and proliferation, and whose level was shown to decrease with skin aging, in human skin organ culture,
- Figure 3: comprises two microscopic images and a graph showing that 2,5-dimethylpyrazine (DMP) increases epidermal expression of Lamin B1, a regulator of cell survival, senescence, and aging, and whose level was shown to decrease with skin aging, in human skin organ culture,
- Figure 4: comprises two microscopic images and a graph showing that 2,5-dimethylpyrazine (DMP) increases epidermal expression of peroxisome proliferator-activated receptor-coactivator-1 α (PGC1a), a transcriptional coactivator that regulates the genes involved in energy metabolism and mitochondrial biogenesis, and whose level was shown to decrease with skin aging, in human skin organ culture,
- Figure 5: comprises two microscopic images and a graph showing that 2,5-dimethylpyrazine (DMP) increases epidermal expression of Sirtuin-1, a NAD-dependent protein deacetylase that links transcriptional regulation directly to intracellular energetics, DNA damage, metabolism, apoptosis and autophagy, and whose level was shown to decrease with skin aging, in human skin organ culture,
- Figure 6: comprises two microscopic images and a graph showing that 2,5-dimethylpyrazine (DMP) increases expression of Collagen 17A in human epidermal keratinocyte culture,
- Figure 7: comprises two microscopic images and a graph showing that 2,5-dimethylpyrazine (DMP) increases expression of Cytokeratin 15 in human epidermal keratinocyte culture,
- Figure 8: comprises two graphs showing that 2,5-dimethylpyrazine (DMP) increases gene expressions of Collagen 17A and Cytokeratin 15 in human epidermal keratinocyte culture,
- Figure 9: comprises two microscopic images and a graph showing that 2,5-dimethylpyrazine (DMP) increases expression of Ki67, a nuclear non-histone protein that is universally expressed among proliferating cells, and whose level was shown to decrease with skin aging, in human epidermal keratinocyte culture,
- Figure 10: comprises two microscopic images and a graph showing that 2,5-dimethylpyrazine (DMP) increases expression of Phospho-Histone H3 Ser10 (pH3S10; phosphorylation at Ser10 of histone H3 is tightly correlated with chromosome condensation during mitosis and cell proliferation, and was shown to decrease with skin aging) in human epidermal keratinocyte culture,
- Figure 11: comprises three microscopic images and a graph showing that triphenyl phosphine oxide (TPPO) prevents the effect of 2,5-dimethylpyrazine (DMP) to increase epidermal expression of Collagen 17A in human skin organ culture,
- Figure 12: comprises three microscopic images and a graph showing that triphenyl phosphine oxide (TPPO) prevents the effect of 2,5-dimethylpyrazine (DMP) to increase epidermal expression of Ki67 in human skin organ culture,
- Figure 13: comprises three microscopic images and a graph showing that triphenyl phosphine oxide (TPPO) prevents the effect of 2,5-dimethylpyrazine (DMP) to increase expression of Collagen 17A in human epidermal keratinocyte culture,
- Figure 14: comprises three microscopic images and a graph showing that triphenyl phosphine oxide (TPPO) prevents the effect of 2,5-dimethylpyrazine (DMP) to increase expression of Cytokeratin 15 in human epidermal keratinocyte culture.

### Examples

### 1. The ex vivo skin organ culture model for assessing skin aging

Samples of human abdominal, breast, temporal, and occipital skin were obtained with written patient consent and under ethical approval from healthy adult human subjects during elective plastic surgery performed for cosmetic reasons. Full-thickness human skin samples were prepared as 4-6 mm punches, placed on filter paper, and cultured at the air-liquid interphase under serum-free conditions in William's E medium supplemented only with penicillin-streptomycin, insulin, hydrocortisone and L-glutamine, as described previously.

For modelling the application of the actives directly to the skin surface (topical application) 2,5-dimethylpyrazine (DMP), a TRPM5 activator was dissolved at 37°C at a final concentration of 12.5 mM in a 50% v/v solution of Isopropanol in PBS containing 1g/mL of Polyethylene glycol 6000. For topical application of triphenylphosphine oxide (TPPO), and TRPM5 inhibitor, a stock was prepared in ethanol and dissolved at 37°C at a final concentration of 1.5 mM in a 50% v/v solution of Isopropanol in PBS containing 1g/mL of Polyethylene glycol 6000. Four µL droplets of this viscous solution containing DMP (12.5 mM), TPPO (1.5 mM), their combination, or ethanol as vehicle control were applied daily (up to 3 days) to the epidermal surface of skin biopsies. For modelling the action of the actives reaching the skin via the bloodstream (systemic application), DMP (12.5 µM) was applied daily (up to 3 days) to the culturing medium.

### 2. In vitro cultures of normal human epidermal keratinocytes for assessing skin aging

Primary adult normal human epidermal keratinocytes (NHEK) were cultured in serum-free Keratinocyte Growth Medium 2 (KGM2) up to passage 6 and were seeded on glass slides at a density of 15-20×10³ cells/cm². The next day, the KGM2 culture medium was changed and supplemented with hereafter indicated compounds, their combination or ethanol as vehicle control.

For application of the active to the medium (systemic application), 2,5-dimethylpyrazine (DMP), a TRPM5 activator, the DMP stock was dissolved at 37°C at a final concentration of 10, 12.5 or 100 nM in KGM2. For triphenylphosphine oxide (TPPO), a TRPM5 inhibitor, a stock was prepared in ethanol and dissolved at 37°C at a final concentration of 50 µM in KGM2. After 24 hours, NHEKs were washed in PBS and then either fixed in 4% formaldehyde for 10 minutes are room temperature (immunofluorescence labeling) or lysed in Buffer RLT containing 1% β-mercaptoethanol (for quantitative RT-qPCR).

### 3. Activation of TRPM5 with 2,5-dimethylpyrazine (DMP)

For the detection of Collagen 17A (an epidermal basal membrane and extracellular matrix component, and whose level was shown to decrease with skin aging) formalin-fixed human skin frozen sections were incubated overnight at 4°C with an anti-Collagen 17A antibody diluted 1:100 in antibody diluent overnight at 4°C. After consecutive rinses with PBS, samples were incubated with Alexa fluorophore-conjugated secondary antibodies diluted 1:500 in antibody diluent containing 2% normal goat serum for 45 minutes at room temperature. After consecutive rinses with PBS, samples were counterstained with DAPI and mounted on glass slides with Fluoromount-G^{®}.

Images were then obtained with a digital microscope (Keyence). Quantitative immunohistomorphometric analysis was performed by assessing the relative fluorescent or color intensity in standardized reference areas with ImageJ (NIH; Bethesda, MD, USA). Briefly, multiple non-consecutive skin sections (at least 280 µm apart from each other) per punch biopsy were stained. Two skin punch biopsies were analyzed per experimental group unless indicated otherwise. Evaluations were performed on multiple different microscopic fields per section. The analysis was performed in the center of the skin biopsy, at least 500 µm away from the edges as these sites show prominent wound-healing processes such as re-epithelialization.

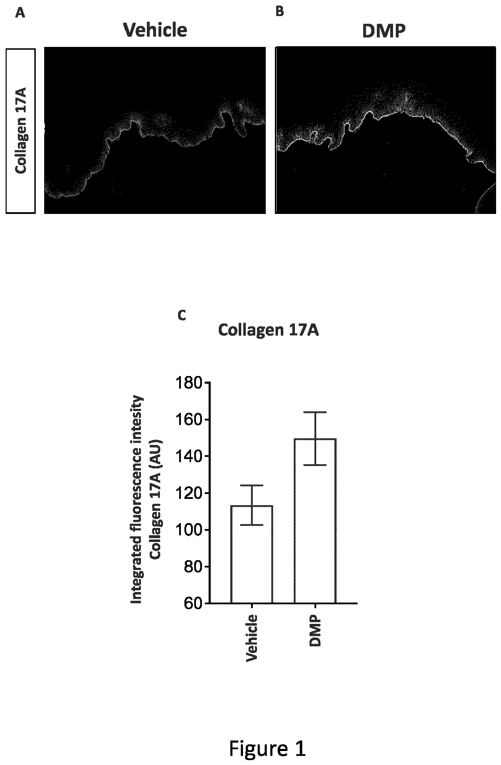

Figure 1 shows representative images (a, b) and the results of the quantitative immunohistomorphometric analysis (c) when performing immunofluorescence visualization of Collagen 17A in skin sections. From Figure 1 it can be identified that the topical application of the TRPM5 agonist 2,5-dimethylpyrazine (DMP, 12.5 mM) markedly increases the expression of Collagen 17A in the basal layers of the epidermis after 3 days in human skin organ culture as compared to vehicle. As the expression level of Collagen 17A decreases with skin aging, this suggests that DMP exerts anti-aging effects.

For the detection of Cytokeratin 15 (a marker of epidermal stem cells and proliferation, and whose level was shown to decrease with skin aging) formalin-fixed human skin frozen sections were incubated overnight at 4°C with an anti-Cytokeratin 15 antibody diluted 1:100 in antibody diluent overnight at 4°C. After consecutive rinses with PBS, samples were incubated with Alexa fluorophore-conjugated secondary antibodies diluted 1:500 in antibody diluent containing 2% normal goat serum for 45 minutes at room temperature. After consecutive rinses with PBS, samples were counterstained with DAPI and mounted on glass slides with Fluoromount-G^{®}. Image acquisition and quantitative immunohistomorphometric analysis were performed similar to as described above.

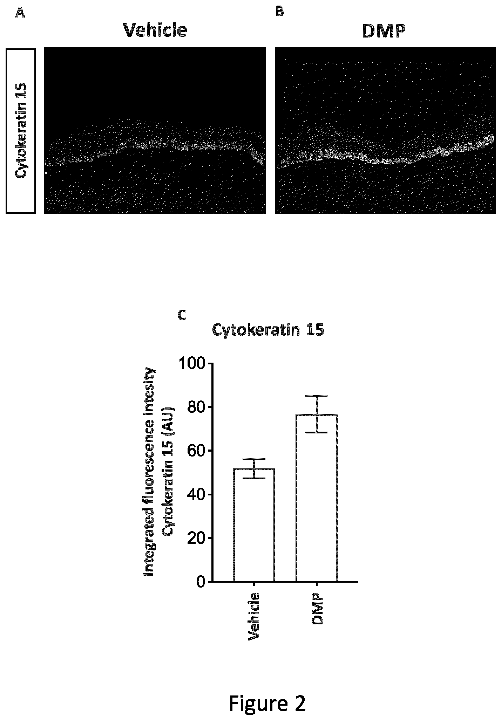

Figure 2 shows representative images (a, b) and the results of the quantitative immunohistomorphometric analysis (c) when performing immunofluorescence visualization of Cytokeratin 15 in skin sections. From Figure 2 it can be identified that the topical application of the TRPM5 agonist 2,5-dimethylpyrazine (DMP, 12.5 mM) markedly increases the expression of Cytokeratin 15 in the basal layers of the epidermis after 3 days in human skin organ culture as compared to vehicle. As the expression level of Cytokeratin 15 decreases with skin aging, this suggests that DMP exerts anti-aging effects.

For the detection of Lamin B1 (a regulator of cell survival, senescence, and aging, and whose reduced level was shown to decrease with skin aging), formalin-fixed human skin frozen sections were permeabilized and blocked with PBS containing 5% BSA, 0.1% Triton and 0.1% Saponin for 10 minutes at room temperature. Samples were then incubated with an anti-Lamin B1 antibody diluted 1:100 in PBS containing 1% BSA, 0.05% Triton and 0.1% Saponin overnight at 4°C. After consecutive rinses with PBS, samples were incubated with Alexa fluorophore-conjugated antibody diluted 1:500 in PBS containing 1% BSA, 0.05% Triton and 0.1% Saponin 45 minutes at room temperature. After consecutive rinses with PBS, samples were counterstained with DAPI and mounted on glass slides with Fluoromount-G^{®}. Image acquisition and quantitative immunohistomorphometric analysis were performed similar to as described above.

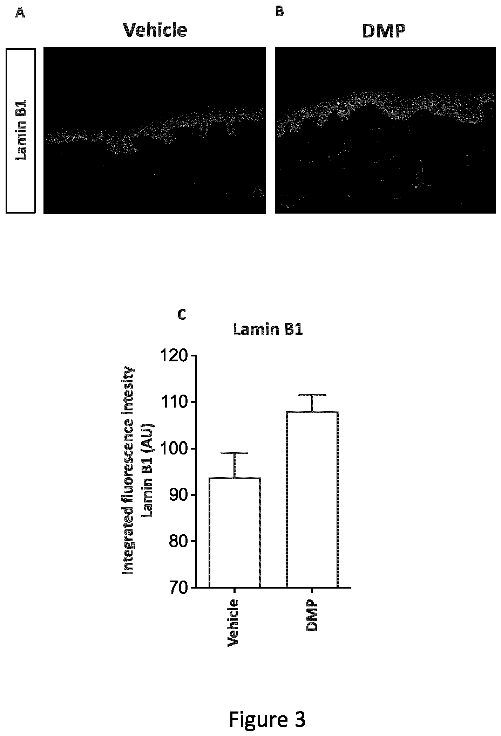

Figure 3 shows representative images (a, b) and the results of the quantitative immunohistomorphometric analysis (c) when performing immunofluorescence visualization of Lamin B1 in skin sections. From Figure 3 it can be identified that the systemic application of the TRPM5 agonist 2,5-dimethylpyrazine (DMP, 12.5 µM) markedly increases the expression of Lamin B1 in (mostly the basal layers of) the epidermis after 3 days in human skin organ culture as compared to vehicle. As the expression level of Lamin B1 decreases with skin aging, this suggests that DMP exerts anti-aging effects.

For the detection of peroxisome proliferator-activated receptor-coactivator-1 α (PGC1α, a transcriptional coactivator that regulates the genes involved in energy metabolism and mitochondrial biogenesis, and whose level was shown to decrease with skin aging), formalin-fixed human skin frozen sections were permeabilized and blocked with Tris-buffered-saline (TBS) containing 0.1% Triton X-100 for 5 minutes at room temperature. Samples were rinsed in TBS, blocked in TBS containing 10% normal goat serum (NGS) for 20 minutes and then incubated with an anti-PGC1α antibody diluted 1:100 in antibody diluent overnight at 4°C. After consecutive rinses with TBS, samples were incubated with fluorophore-conjugated secondary antibody diluted 1:200 in antibody diluent containing 2% NGS. After consecutive rinses with PBS, samples were counterstained with DAPI and mounted on glass slides with Fluoromount-G^{®}. Image acquisition and quantitative immunohistomorphometric analysis were performed similar to as described above.

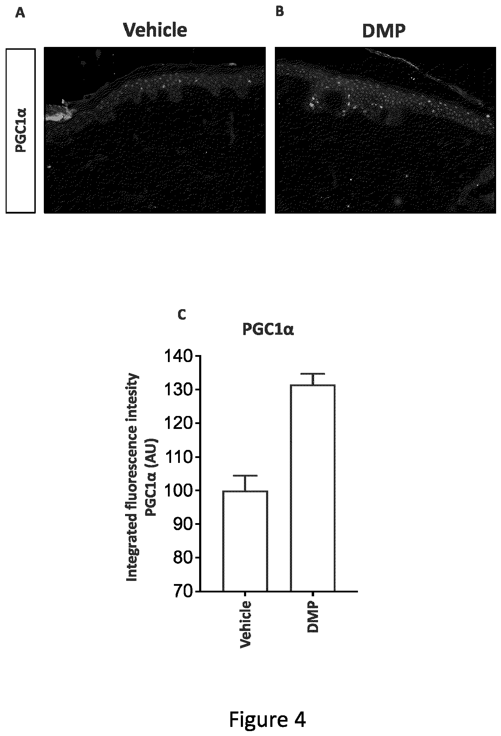

Figure 4 shows representative images (a, b) and the results of the quantitative immunohistomorphometric analysis (c) when performing immunofluorescence visualization of PGC1a in skin sections. From Figure 4 it can be identified that the systemic application of the TRPM5 agonist 2,5-dimethylpyrazine (DMP, 12.5 µM) markedly increases the expression of PGC1a in the epidermis after 3 days in human skin organ culture as compared to vehicle. As the expression level of PGC1α decreases with skin aging, this suggests that DMP exerts anti-aging effects.

For the detection of Sirtuin-1 (a NAD-dependent protein deacetylase that links transcriptional regulation directly to intracellular energetics, DNA damage, metabolism, apoptosis and autophagy, and whose level was shown to decrease with skin aging), acetone-fixed human skin frozen sections were washed with Tris-buffered-saline (TBS). Endogenous peroxidase activity was blocked by incubation in TBS containing 3% H₂O₂ for 15 minutes at room temperature. Endogenous biotin was blocked using the Avidin/Biotin Blocking Kit according to the manufacturer's instructions. Samples were rinsed in TBS and incubated with an anti-Sirtuin 1 antibody diluted 1:400 in antibody diluent overnight at 4°C. After consecutive rinses with TBS, samples were incubated with a biotinylated anti-rabbit secondary antibody diluted 1:200 in antibody diluent. After consecutive rinses with TBS, samples were incubated with Avidin-Biotin Peroxidase for 30 minutes at 37°C. After consecutive rinses with TBS, samples were incubated with AEC Peroxidase Substrate according to manufacturer's instructions. Samples were rinsed in TBS for 5 minutes and embedded in Faramount. Image acquisition and quantitative immunohistomorphometric analysis were performed similar to as described above.

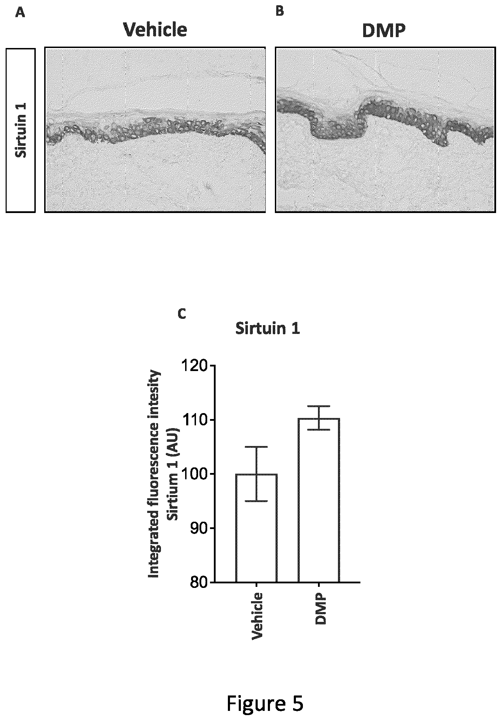

Figure 5 shows representative images (a, b) and the results of the quantitative immunohistomorphometric analysis (c) when performing immunohistochemical visualization of Sirtuin 1 in skin sections. From Figure 5 it can be identified that the systemic application of the TRPM5 agonist 2,5-dimethylpyrazine (DMP, 12.5 µM) markedly increases the expression of Sirtuin 1 in the epidermis after 3 days in human skin organ culture as compared to vehicle. As the expression level of Sirtuin 1 decreases with skin aging, this suggests that DMP exerts anti-aging effects.

For the detection of Collagen 17A in cell cultures, formalin-fixed NHEKs were permeabilized in PBS containing 0.05% Triton^{™} X-100 for 10 minutes, rinsed in PBS and then blocked in PBS containing 10% normal goat serum (NGS) for 20 minutes. Next, samples were incubated with an anti-Collagen 17A diluted 1:100 in antibody diluent overnight at 4°C. After consecutive rinses with PBS, samples were incubated with Alexa fluorophore-conjugated secondary antibodies diluted 1:500 in antibody diluent containing 2% NGS for 45 minutes at room temperature. After consecutive rinses with PBS, samples were counterstained with DAPI and mounted on glass slides with Fluoromount-G^{®}.

For the analysis on NHEKs, images were then obtained with a digital microscope (Keyence). Quantitative immunohistomorphometric analysis was performed by assessing the number of positive cells or the relative fluorescence intensity in standardized reference areas with ImageJ (NIH; Bethesda, MD, USA). Briefly, for the evaluation of Collagen 17A, the relative target-specific intensity was measured in 14-20 cells per microscopic field. For each parameter 14-20 microscopic fields were evaluated.

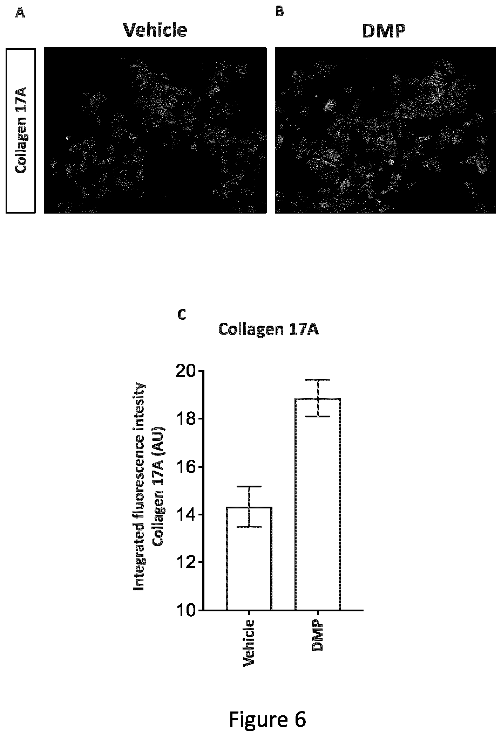

Figure 6 shows representative images (a, b) and the results of the quantitative immunohistomorphometric analysis (c) when performing immunofluorescence visualization of Collagen 17A in NHEK cultures. From Figure 6 it can be identified that the application of the TRPM5 agonist 2,5-dimethylpyrazine (DMP, 12.5 nM) markedly increases the expression of Collagen 17A in the NHEKs after 24 hrs in culture as compared to vehicle. This confirms the findings on human organ cultured skin samples (see Figure 1) that DMP exerts anti-aging effects.

For the detection of Cytokeratin 15 in cell cultures, formalin-fixed NHEKs were permeabilized in PBS containing 0.05% Triton^{™} X-100 for 10 minutes, rinsed in PBS and then blocked in PBS containing 10% normal goat serum (NGS) for 20 minutes. Next, samples were incubated with an anti-Cytokeratin 15 diluted 1:100 in antibody diluent overnight at 4°C. After consecutive rinses with PBS, samples were incubated with Alexa fluorophore-conjugated secondary antibodies diluted 1:500 in antibody diluent containing 2% NGS for 45 minutes at room temperature. After consecutive rinses with PBS, samples were counterstained with DAPI and mounted on glass slides with Fluoromount-G^{®}.

For the analysis on NHEKs, images were then obtained with a digital microscope (Keyence). Quantitative immunohistomorphometric analysis was performed by assessing the number of positive cells or the relative fluorescence intensity in standardized reference areas with ImageJ (NIH; Bethesda, MD, USA). Briefly, for the evaluation of Cytokeratin 15, the relative target-specific intensity was measured in 14-20 cells per microscopic field. For each parameter 14-20 microscopic fields were evaluated.

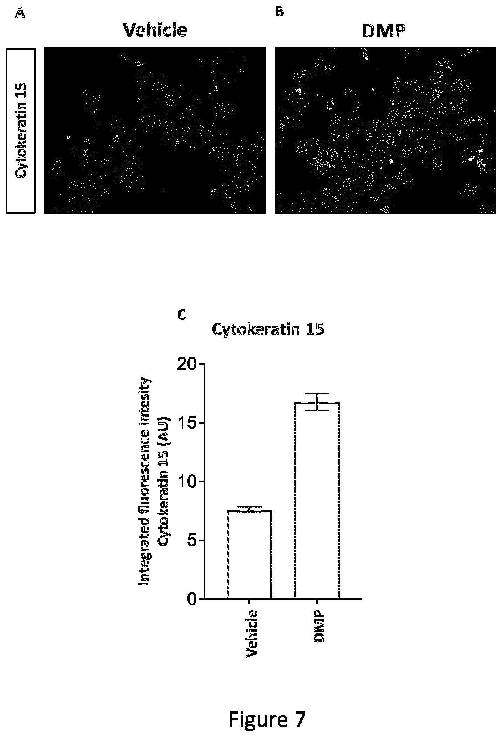

Figure 7 shows representative images (a, b) and the results of the quantitative immunohistomorphometric analysis (c) when performing immunofluorescence visualization of Cytokeratin 15 in NHEK cultures. From Figure 7 it can be identified that the application of the TRPM5 agonist 2,5-dimethylpyrazine (DMP, 12.5 nM) markedly increases the expression of Cytokeratin 15 in the NHEKs after 24 hrs in culture as compared to vehicle. This confirms the findings on human organ cultured skin samples (see Figure 2) that DMP exerts anti-aging effects.

For the assessment of Collagen 17A and Cytokeratin 15 specific mRNA transcripts (*COL17A*, *KRT15*) by quantitative RT-qPCR technology, RNA was isolated from cells lysed in Buffer RLT containing 1% β-mercaptoethanot using the RNeasy Mini Kit according to the manufacturer's instructions. RNA purity and concentrations were determined using the BioDrop. For reverse transcription, 500 ng of RNA of sample was used for subsequent cDNA synthesis using the Tetro^{™} cDNA Synthesis Kit according to the manufacturer's instructions. Real-time quantitative polymerase chain reaction (qRT-PCR) was run in triplicate using TaqMan^{™} Fast Advanced Master Mix and TaqMan Gene Expression Assays on the QuantStudio. Real-time quantification plots and Ct values were collected and stored by the qPCRsoft2.1 software. The amount of the transcripts was normalized to those of the housekeeping gene GAPDH by the ΔΔCT method.

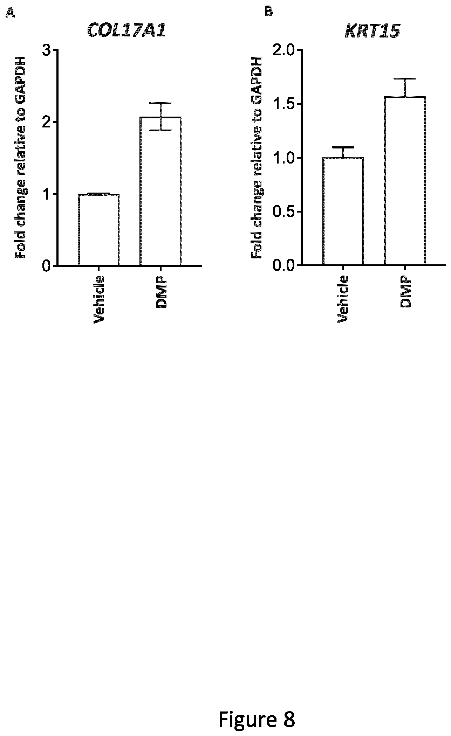

Figure 8 shows the results of the RT-qPCR analysis in NHEK cultures. From Figure 8 it can be identified that the application of the TRPM5 agonist 2,5-dimethylpyrazine (DMP) (a, 10 nM; b, 100 nM) markedly increases the expression of *COL17A1* (a) and *KRT15* (b) in the NHEKs after 24 hrs in culture as compared to vehicle. This confirms the immunohistomorphometric findings on human organ cultured skin samples and NHEKs (see Figures 1, 2, 6, 7) that DMP exerts anti-aging effects.

For the detection Ki67 (which is a nuclear non-histone protein that is universally expressed among proliferating cells) in cell cultures, formalin-fixed NHEKs were permeabilized and blocked in PBS containing 5% normal goat serum and 0.3% Triton^{™} X-100 for 30 minutes. After rinsing with PBS, samples were incubated with an anti-Ki67 antibody diluted 1:800 in PBS containing 1% BSA and 0.05% Triton^{™} X-100 overnight at 4°C. After consecutive rinses with PBS, samples were incubated with Alexa fluorophore-conjugated secondary antibodies diluted 1:500 in PBS containing 1% BSA and 0.05% Triton^{™} X-100 for 45 minutes at room temperature. After consecutive rinses with PBS, samples were counterstained with DAPI and mounted on glass slides with Fluoromount-G^{®}.

For the analysis on NHEKs, images were then obtained with a digital microscope (Keyence). Quantitative immunohistomorphometric analysis was performed by assessing the number of positive cells or the relative fluorescence intensity in standardized reference areas with ImageJ (NIH; Bethesda, MD, USA). Briefly, for the evaluation of Ki67, the number of cells with positive nuclear staining per field was counted and normalized to the total number of DAPI positive nuclei. For each parameter 14-20 microscopic fields were evaluated.

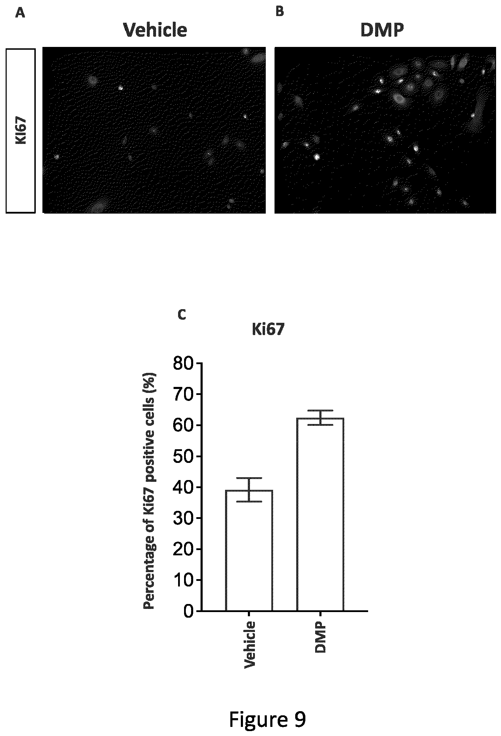

Figure 9 shows representative images (a, b) and the results of the quantitative immunohistomorphometric analysis (c) when performing immunofluorescence visualization of Ki67 in NHEK cultures. From Figure 9 it can be identified that the application of the TRPM5 agonist 2,5-dimethylpyrazine (DMP, 100 nM) markedly increases the number of Ki67 positive (i.e. proliferating) NHEKs after 24 hrs in culture as compared to vehicle. As the rate of proliferation of human epidermal keratinocytes decreases with aging, this suggests that DMP exerts anti-aging effects.

For the detection of Phospho-Histone H3 Ser10 (pH3S10) (phosphorylation at Ser10 of histone H3 is tightly correlated with chromosome condensation during mitosis and cell proliferation) in cell cultures, formalin-fixed NHEKs were permeabilized and blocked in PBS containing 5% normal goat serum and 0.3% Triton^{™} X-100 for 30 minutes. After rinsing with PBS, samples were incubated with an anti-pH3S10 antibody diluted 1:200 in PBS containing 1% BSA and 0.05% Triton^{™} X-100 overnight at 4°C. After consecutive rinses with PBS, samples were incubated with Alexa fluorophore-conjugated secondary antibody diluted 1:500 in PBS containing 1% BSA and 0.05% Triton^{™} X-100 for 45 minutes at room temperature. After consecutive rinses with PBS, samples were counterstained with DAPI and mounted on glass slides with Fluoromount-G^{®}.

For the analysis on NHEKs, images were then obtained with a digital microscope (Keyence). Quantitative immunohistomorphometric analysis was performed by assessing the number of positive cells or the relative fluorescence intensity in standardized reference areas with ImageJ (NIH; Bethesda, MD, USA). Briefly, for the evaluation of pH3S10, the number of cells with positive nuclear staining per field was counted and normalized to the total number of DAPI positive nuclei. For each parameter 14-20 microscopic fields were evaluated.

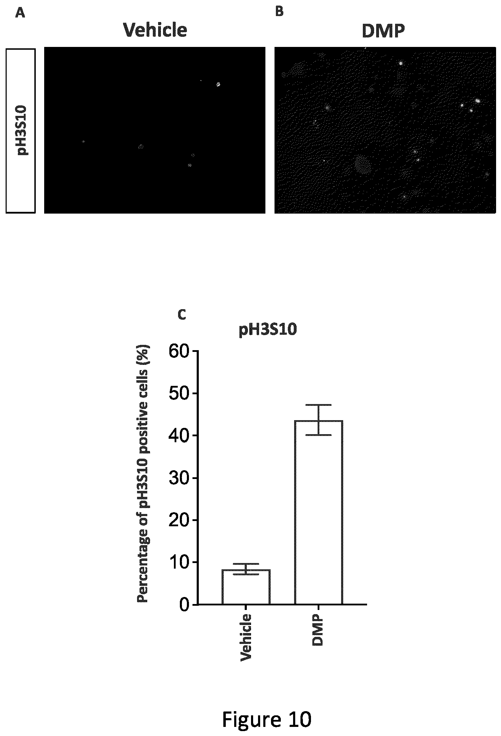

Figure 10 shows representative images (a, b) and the results of the quantitative immunohistomorphometric analysis (c) when performing immunofluorescence visualization of pH3S10 in NHEK cultures. From Figure 10 it can be identified that the application of the TRPM5 agonist 2,5-dimethylpyrazine (DMP, 100 nM) markedly increases the number of pH3S10 positive (i.e. dividing and proliferating) NHEKs after 24 hrs in culture as compared to vehicle. As the rate of cell division (hence proliferation) of human epidermal keratinocytes decreases with aging, this suggests that DMP exerts anti-aging effects.

### 4. Inactivation of TRPM5 with Triphenyl phosphine oxide (TPPO)

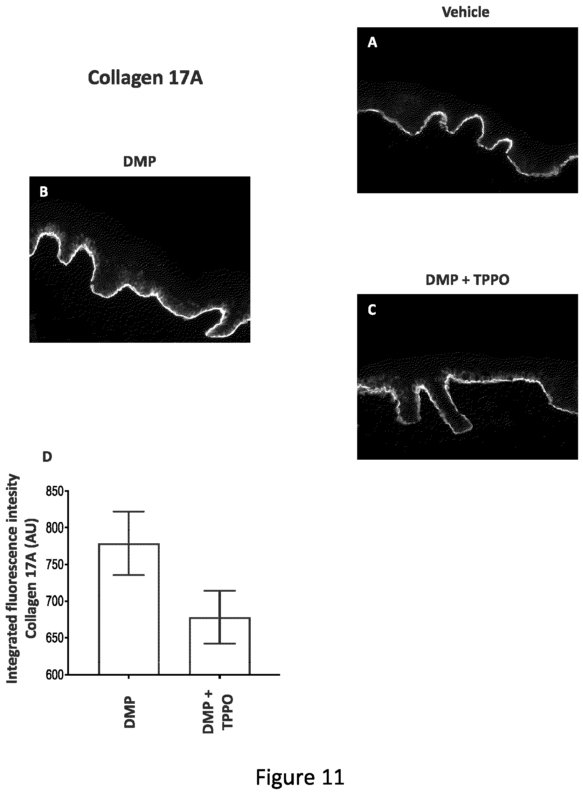

Figures 11 shows representative images (a, b, c) and the results of the quantitative immunohistomorphometric analysis (d) when performing immunofluorescence visualization of Collagen 17A in skin sections. From Figure 11 it can be identified that the topically applied TRPM5 antagonist triphenyl phosphine oxide (TPPO, 1.5 mM) prevented the effect of the topically applied TRPM5 agonist 2,5-dimethylpyrazine (DMP, 12.5 mM) to upregulate the expression of Collagen 17A after 3 days in human skin organ culture as compared to vehicle. This suggests that the anti-aging effect of DMP is mediated by TRPM5.

For the detection of Ki67, formalin-fixed human skin frozen sections were blocked with PBS containing 10% normal goat serum (NGS) for 30 minutes at room temperature and then incubated with an anti-Ki67 antibody diluted 1:800 in PBS containing 2% NGS overnight at 4°C. After consecutive rinses with PBS, samples were incubated with secondary antibodies, α-rabbit Alexa 488 fluorophore-conjugated diluted 1:400 and a-mouse Rhodamine conjugated diluted 1:200, in PBS containing 2% NGS for 45 minutes at room temperature. After consecutive rinses with PBS, samples were counterstained with DAPI and mounted on glass slides with Fluoromount-G^{®}. Image acquisition and quantitative immunohistomorphometric analysis were performed similar to as described above.

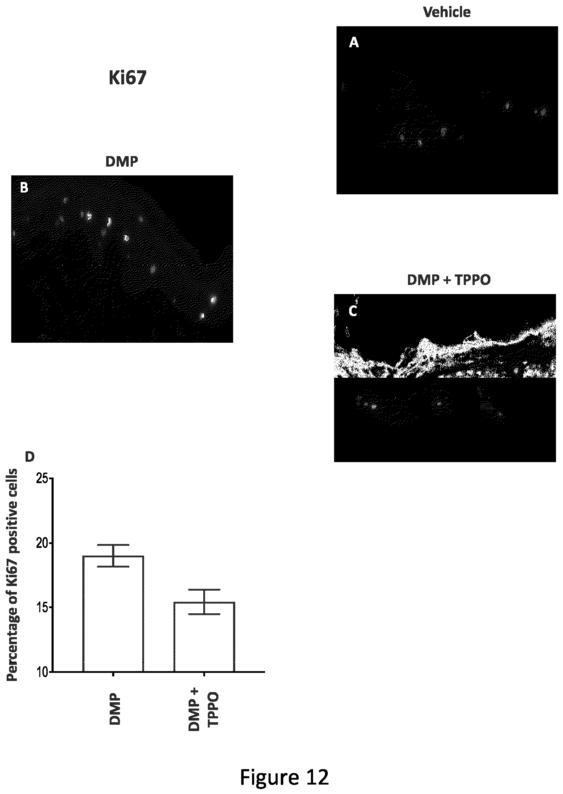

Figures 12 shows representative images (a, b, c) and the results of the quantitative immunohistomorphometric analysis (d) when performing immunofluorescence visualization of Ki67 in skin sections. From Figure 12 it can be identified that the topically applied TRPM5 antagonist triphenyl phosphine oxide (TPPO, 1.5 mM) prevented the effect of the topically applied TRPM5 agonist 2,5-dimethylpyrazine (DMP, 12.5 mM) to upregulate the expression of Ki67 after 3 days in human skin organ culture as compared to vehicle. This suggests that the anti-aging effect of DMP is mediated by TRPM5.

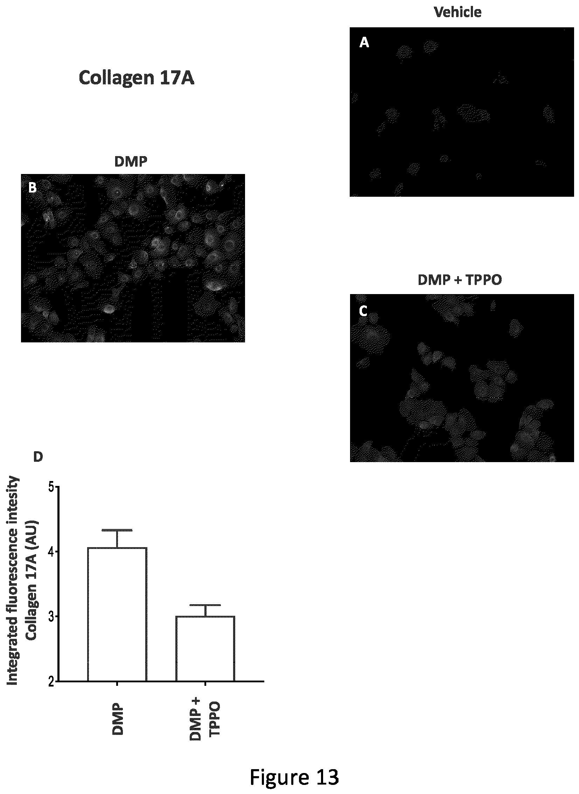

Figures 13 shows representative images (a, b, c) and the results of the quantitative immunohistomorphometric analysis (d) when performing immunofluorescence visualization of Collagen 17A in NHEK cultures. From Figure 13 it can be identified that the TRPM5 antagonist triphenyl phosphine oxide (TPPO, 50 µM) prevented the effect of the TRPM5 agonist 2,5-dimethylpyrazine (DMP, 100 nM) to upregulate the expression of Collagen 17A after 24 hrs in culture as compared to vehicle. This suggests that the anti-aging effect of DMP is mediated by TRPM5.

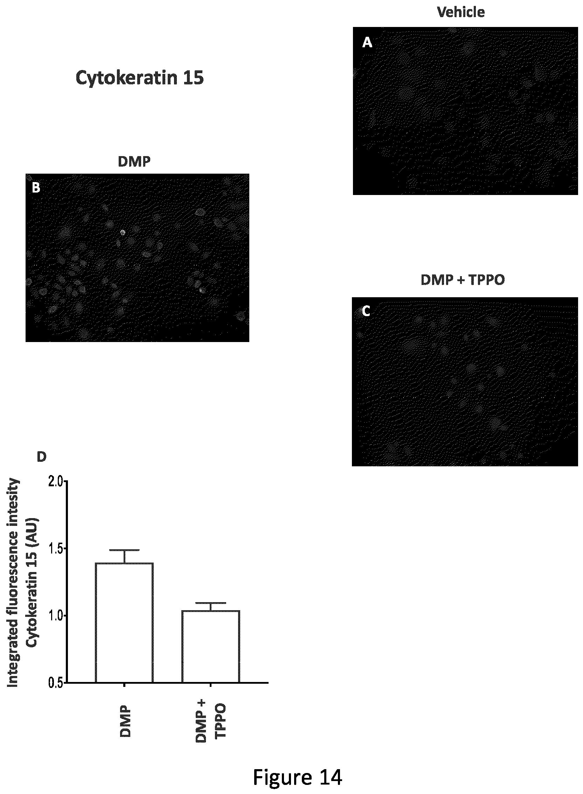

Figures 14 shows representative images (a, b, c) and the results of the quantitative immunohistomorphometric analysis (d) when performing immunofluorescence visualization of Cytokeratin 15 in NHEK cultures. From Figure 14 it can be identified that the TRPM5 antagonist triphenyl phosphine oxide (TPPO, 50 µM) prevented the effect of the TRPM5 agonist 2,5-dimethylpyrazine (DMP, 100 nM) to upregulate the expression of Cytokeratin 15 after 24 hrs in culture as compared to vehicle. This suggests that the anti-aging effect of DMP is mediated by TRPM5.

## Claims

1. Active agent for use in the treatment of skin aging, particularly for use in the treatment of premature or pathological skin aging and pathological skin conditions related to or induced by skin aging, wherein said active agent activates, enhances or positively modulates the cellular response of the transient receptor potential ion channel TRPM5 or increases the expression of said ion channel.

2. Active agent for use in the treatment of skin aging according to claim 1, wherein the active agent is an agonist of the transient receptor potential ion channel TRPM5 for use in the treatment of skin aging.

3. Active agent for use in the treatment of skin aging according to one of the claims 1 to 2, wherein said active agent is selected from any one of the TRPM5 activating agonists dimethylpyrazine, dimethylethylpyrazine, 2-heptanone, SID2848719 (CAS number 702636-90-6), rutamarin, xanthotoxin, isopimpinellin, carbachol, 3-deoxyglucosone, (E)-N-(3,4dimethoxybenzylidene)-2-naphthalene-1-yl) acetohydrazide or a combination thereof, or an aptamer binding to TRPM5 and activating or enhancing said ion channel to produce a cellular response.

4. Active agent for use in the treatment of skin aging according to one of the claims 1 to 3, wherein the treatment is
a) for treating of skin aging of any form **characterized by** thin, dry, fragile and sensitive skin, fine or course wrinkles, gradual atrophy that affects both the epidermis and the dermis, loss of elasticity and laxity, decrease of skin water content, rough texture, impaired and delayed wound regeneration, and/or
b) for treating aging-associated skin disease selected rosacea (redness, pimples, swelling, and small and superficial dilated blood vessels affecting especially the face), asteatotic eczema (**characterized by** dry, itchy, and cracked skin), nummular eczema (features coin-shaped itchy, reddened patches), seborrheic dermatitis, skin infections, actinic keratosis (precancerous skin lesion linked to chronic UV irradiation), and benign (non-cancerous) as well as malignant (basal cell carcinoma, squamous cell carcinoma) skin tumors.

5. Use of an active agent according to one of the claims 1 to 4 as a cosmetic for the non-therapeutical treatment of skin aging and associated conditions.

6. Cosmetic or medical skin anti-aging composition comprising at least one active agent according to one of the claims 1 to 4 and at least one auxiliary agent selected from the group consisting of carriers, excipients, adjuvants, diluents, and disintegrants.

7. Cosmetic or medical skin anti-aging composition for according to claim 6, wherein the auxiliary agent is selected from the group consisting of liposomes, nanoparticles, carboxymethyl cellulose, hydroxyethyl cellulose, mineral oil, petrolatum, glycerine, polysorbate 80, hydroxyethyl starch, dextran, and polyethylene glycol.

8. Cosmetic or medical skin anti-aging composition according to claim 6 or claim 7, further comprising at least one other active agent being effective in the treatment of skin aging.

9. Cosmetic or medical skin anti-aging composition according to one of the claims 6 to 8, wherein the composition is formulated in the form of an ointment, a lotion, a cream, a gel, a solution, a spray, a plaster or a sustained release plaster.

10. Non-therapeutic method of skin aging control, wherein an effective amount of at least one active agent that activates, enhances, or modulates the cellular response of the transient receptor potential ion channel TRPM5 or increases the expression of said ion channel is administered to a subject.
